# EUROPEAN PATENT SPECIFICATION

(11) **EP 1 637 145 B1**
(45) Date of publication and mention of the grant of the patent: **15.05.2013**
(21) Application number: 04745832.8
(22) Date of filing: 07.06.2004
(51) Int. Cl.: A61K 35/14, A61L 15/44

(54) **MATERIAL PROMOTING WOUND HEALING**
MATERIAL ZUR FÖRDERUNG DER WUNDHEILUNG
MATIERE FACILITANT LA GUERISON D'UNE PLAIE

(30) Priority: 06.06.2003 JP 2003161887
(43) Date of publication of application: 22.03.2006
(73) Proprietor: Asahi Kasei Medical Co., Ltd., Tokyo 101-8101 (JP)
(72) Inventor: IWAMOTO, Ushio, Oita-shi, Oita 8700253 (JP); TOKUSHIMA, Yasuo, Oita-shi, Oita 8700302 (JP); KITAGUCHI, Nobuya, 4170001 (JP)
(74) Representative: von Kreisler Selting Werner
(86) International application number: PCT/JP2004/008254
(87) International publication number: WO 2004/108146

(56) References cited:
- EP-A- 0 502 213
- WO-A1-01/91880
- WO-A1-96/27397
- WO-A1-99/58172
- JP-A- 5 043 453
- JP-A- 6 500 802
- JP-A- 7 059 840
- JP-A- 7 507 558
- JP-A- 8 224 293
- JP-A- 11 239 609
- JP-A- 62 501 628
- JP-A- 2001 204 807
- JP-A- 2001 508 807
- JP-A- 2002 047 299
- JP-A- 2002 531 532
- JP-A- 2003 010 301
- JP-A- 2003 524 590
- US-A- 4 416 777
- US-A- 5 510 102
- US-A- 6 049 026
- NG K W ET AL: "In vitro characterization of natural and synthetic dermal matrices cultured with human dermal fibroblasts" BIOMATERIALS, ELSEVIER SCIENCE PUBLISHERS BV., BARKING, GB, vol. 25, no. 14, 1 June 2004 (2004-06-01), pages 2807-2818, XP004489029 ISSN: 0142-9612
- KALKA C, ET AL: 'Transplantation of ex vivo expanded endothelial progenitor cells for therapeutic neovascularization' PROC. NATL. ACAD. SCI. USA vol. 97, no. 7, 2000, pages 3422 - 3427, XP002204951
- ZHAO Y, ET AL: 'A human peripheral blood monocyte-derived subset acts as pluripotent stem cells' PROC. NATL. ACCAD. SCI. USA vol. 100, no. 5, 04 March 2003, pages 2426 - 2431, XP002979380
- VALBONESI M, ET AL: 'The role of autologous fibrin-platelet glue in plastic surgery: a preliminary report' INT. J. ART. ORGANS. vol. 25, no. 4, 2002, pages 334 - 338, XP009025485

## Description

### Technical Field

The present invention relates to a material for use as a wound-healing promoting material, and the use of a sheet-like porous body having on its surface at least leukocytes for the production of a material for use as a wound-healing promoting material.

### Background Art

In recent years, mechanisms involved in wound healing have progressively become elucidated. As one example of such mechanisms, it is known that a variety of growth factors produced by cells in blood such as leukocytes or platelets play a key role in tissue regeneration.

The term "growth factor" used herein refers to a factor that directly or indirectly regulates a wide variety of phenomena involved in wound healing, ranging from blood coagulation, migration of inflammatory cells, growth of fibroblasts, synthesis of extracellular matrix, neoangiogenesis or neovascularization, to reconstitution thereof, within a complicated intercellular network correlation. For example, functions of the platelet-derived growth factor (PDGF) that had been discovered as one type of platelet-derived growth factor have been elucidated. Specifically, PDGF promotes the migration and the growth of fibroblasts or smooth muscle cells, potently influences monocyte or neutrophil chemotaxis, and promotes collagen and collagenase synthesis in fibroblasts. Pierce et al. made wounds in rabbit ears surgically, which would reach the cartilage, and administered PDGF-BB to rabbits. As a result, the wounds of rabbits to which PDGF-BB had been administered were found to heal faster than the wounds of rabbits to which PDGF-BB had not been administered (J. Cell Biochem. 1991, 45: 319-326). As the name implies, a large number of PDGFs are contained in platelets. Also, PDGFs are produced by cells such as macrophages, vascular endothelial cells, or smooth muscle cells. As growth factors involved in wound healing, for example, PDGF-BB as well as growth factors such as fibroblast growth factors (FGF), vascular endothelial growth factors (VEGF), transforming growth factors-β (TGF-β) and epidermal growth factors (EGF) have been discovered. Some thereof have become clinically applied.

As other wound-healing mechanisms, several mechanisms have been discovered from the viewpoint of involvement of blood cells in wound healing. For example, Kalka et al. discloses a process whereby mononuclear cells in human peripheral blood were cultured *in vitro,* the cultured cells were then transplanted to the ischemic areas of nude rats that had been subjected to luminal narrowing, and the transplanted cells took hold and generated new blood vessels to heal the ischemic areas. Thus, effects of the peripheral blood-derived leukocytes on wound healing are expected from the viewpoint of the direct involvement thereof in neovascularization in addition to the aforementioned growth factor production (PNAS. 2000, 97: 3422-3427). Further, Zhao et al. discloses a method whereby a monocytic fraction in the human peripheral blood is cultured *in vitro* using a variety of growth factors and is differentiated into cells such as epidermic cells, vascular endothelial cells, hepatic cells and nerve cells. Accordingly, it is expected that a combination of cells and growth factors would yield further wound-healing effects (PNAS. 2003, 100: 2426-2431).

For wound healing, a specific growth factor or cell is administered to a patient, and such administration is primarily carried out systemically or locally. However, it is suggested that systemic administration of an excess amount of a growth factor, such as VEGF may cause low blood pressure, and systemic administration of an excess amount of bFGF may cause nephrotoxicity. Thus, the dose thereof had to be determined with extra care. In contrast, local administration of growth factors or cells is considered suitable; however, it is difficult to locally maintain growth factor concentration for a given period of time. Since the communication between cells involved in wound healing is a complicated system that involves a wide variety of factors as mentioned above, the effects of healing resulting from the administration of a single type of growth factor is often insufficient. Thus, it is considered that administration of various types of growth factors involved in wound healing is necessary. Gene therapy is another example of a method for local administration, but the safety of gene therapy has not yet been sufficiently assured at present.

Local administration of certain cells is considered to be more effective than local administration of growth factors, and various techniques therefor have been studied. For example, platelets that produce growth factors are separated from blood and concentrated via centrifugation, fibrinogens and thrombins are added thereto to prepare platelet glue, and the thus prepared platelet glue is applied to the wound site (Int. J. Artif. Organs. 2002, 25(4): 334-8). This technique had problems that it was time-consuming and laborious in respect of preparation, and the mechanical strength of the resultant glue was insufficient. JP Patent Publication (Unexamined) No. 2001-204807 discloses a medical material which comprises a gel comprising cells such as fibroblasts in a skeleton consisting of the molded product of polymer materials for the purpose of enhancing the mechanical strength of such gel. This medical material is primarily used as an artificial skin, and the gel does not contain leukocytes, platelets, or the like. Thus, the effects of promoting wound healing could not be expected. Also, cells in the gel are fixed in the gel. Thus, there was a problem that even if such cells produced some type of growth factor, it would take a long time for such factor to reach the wound site. US Patent No. 6,049,026 discloses a kit for allowing connective tissue precursor cells in bone marrow to adhere to the surface of the substrate as grafts, and platelets are further allowed to adhere in order to promote the growth of the grafts. This invention is, however, primarily associated with bone grafts, and is not intended to promote the healing of wounds or the growth of cells at the wound sites of organisms. Accordingly, adherence of leukocytes to a graft is not disclosed at all.

As described above, a material for (promoting) wound healing that has utilized blood cells having the wound-healing effects in a more effective way has not yet been known in the art of wound healing. Therefore, technical innovation concerning such material has been awaited.

JP-A-62-0501628 discloses a pharmaceutical composition comprising platelet-derived factor. The composition is gelatinized to be a body tissue adhesive which is prepared at the target site to be treated.

EP-A-0502213 discloses a filter for leukocyte removal from blood. The devices are *suitable* as wound-healing promoting material.

US-A-4,416,777 discloses a leukocyte-separating material made of fibrous polyacrylnitrile fibers. The filter material in use contains leukocytes. The devices are *suitable* as wound-healing promoting material.

### Disclosure of the Invention

The object to be solved by the present invention is to overcome the aforementioned drawbacks of the prior art. Specifically, the object to be solved by the present invention is to provide a means for utilizing cells that have effects on wound healing as wound-healing promoting materials by efficiently concentrating such cells within a short period of time.

The present inventors have conducted concentrated studies in order to attain the above object. As a result, they discovered that a sheet-like porous material having on its surface leukocytes can improve the growth of fibroblasts, and allow them to produce growth factors involved in wound healing, thereby promoting wound healing. This has led to the completion of the present invention.

Thus, the present invention relates to the following:
(1) A material for use as a wound-healing promoting material which comprises a sheet-like porous body having on its surface at least leukocytes.
(2) The material according to (1) which has a fibroblast-proliferating potency.
(3) The material according to any of (1) or (2), wherein the sheet-like porous body has a thickness of 0.01 mm to 3 mm.
(4) The material according to any of (1) to (3), wherein the shape of the sheet-like porous body can be altered in accordance with the shape of the wound site.
(5) The material according to (4), wherein the sheet-like porous body is made of a nonwoven fabric having a fiber diameter of 0.3 µm to 50 µm and a bulk density of 0.05 g/cm³ to 0.5 g/cm³, or a sponge construct having an average pore diameter of 1.0 µm to 40 µm.
(6) The material according to any (1) to (5), wherein the sheet-like porous body is made of a natural or synthetic polymer.
(7) The material according to (6), wherein the sheet-like porous body is made of a biodegradable material.
(8) The material according to any of (1) to (7), wherein the leukocytes are derived from the peripheral blood, bone marrow fluid, or umbilical cord blood.
(9) The material according to any of (1) to (8), wherein the leukocytes are mature cells.
(10) The material according to (8) or (9), wherein the leukocytes are derived from autologous blood.
(11) The material according to any of (1) to (10), wherein the sheet-like porous body has a leukocyte density of 6.0x 10⁶ cells/cm³ or higher.
(12) The material according to any of (1) to (11), wherein the sheet-like porous body comprises fibroblasts incorporated therein.
(13) The material according to any of (1) to (12), wherein the sheet-like porous body comprises fibrins.
(14) Use of a sheet-like porous body having on its surface at least leukocytes for the production of a material for use as a wound-healing promoting material.
(15) The use according to (14), wherein the wound-healing promoting material which has a fibroblast-proliferating potency.
(16) The use of (14) or (15), wherein the sheet-like porous body has a thickness of 0.01 mm to 3 mm.
(17) The use according to any of (14) to (16), wherein the shape of the sheet-like porous body can be altered in accordance with the shape of the wound site.
(18) The use according to (17), wherein the sheet-like porous body is made of a nonwoven fabric having a fiber diameter of 0.3 µm to 50 µm and a bulk density of 0.05 g/cm³ to 0.5 g/cm³, or a sponge construct having an average pore diameter of 1.0 µm to 40 µm.
(19) The use according to any of (14) to (18), wherein the sheet-like porous body is made of a natural or synthetic polymer.
(20) The use according to (19), wherein the sheet-like porous body is made of a biodegradable material.
(21) The use according to any of (14) to (20), wherein the leukocytes are derived from the peripheral blood, bone marrow fluid, or umbilical cord blood.
(22) The use according to any of (14) to (21), wherein the leukocytes are mature cells.
(23) The use according to (21) or (22), wherein the leukocytes are derived from autologous blood.
(24) The use according to any of (14) to (23), wherein the sheet-like porous body has a leukocyte density of 6.0 x 10⁶ cells/cm³ or higher.
(25) The use according to any of (14) to (24), wherein the sheet-like porous body comprises fibroblasts incorporated therein.
(26) The use according to any of (14) to (25), wherein the sheet-like porous body comprises fibrins.

Also disclosed but not part of the invention is:
(a) A device for preparing a wound-healing promoting material which comprises an openable liquid-tight container equipped with an inlet and an outlet for liquid injection and discharge, wherein a sheet-like porous body is positioned in a manner such that the interior of the container is divided into two sections, and the one end is connected to the inlet and the other end is connected to the outlet.
(b) The device for preparing a wound-healing promoting material according to (a), which is a soft device prepared by sandwiching the sheet-like porous body between flexible resin sheets and welding them or causing them to adhere to each other, wherein the sheet-like porous body therein can be exposed or removed therefrom by peeling of the flexible resin sheets.
(c) The device for preparing a wound-healing promoting material according to (b), which is a flat plate.
(d) The device for preparing a wound-healing promoting material according to (a), which is a hard device equipped with a means of sealing, wherein the sheet-like porous body therein can be exposed or removed therefrom by release of the means of sealing.
(e) The device for preparing a wound-healing promoting material according to (d), which is a cylinder.
(f) The device for preparing a wound-healing promoting material according to (e), wherein the sheet-like porous body is wound into a roll and is contained.
(g) The device for preparing a wound-healing promoting material according to any of (a) to (f), wherein the sheet-like porous body has a thickness of 0.01 mm to 3 mm.
(h) The device for preparing a wound-healing promoting material according to any of (a) to (f), wherein the sheet-like porous body is made of a nonwoven fabric.
(i) The device for preparing a wound-healing promoting material according to (h), wherein the nonwoven fabric has a fiber diameter of 0.3 µm to 50 µm and a bulk density of 0.05 g/cm³ to 0.5 g/cm³.
(j) The device for preparing a wound-healing promoting material according to any of (a) to (g), wherein the sheet-like porous body is a sponge construct. any of (a) to (g), wherein the sheet-like porous body is a sponge construct.
(k) The device for preparing a wound-healing promoting material according to (g), wherein the sponge construct has an average pore diameter of 1.0 µm to 40 µm.
(l) The device for preparing a wound-healing promoting material according to any of (a) to (k), wherein the sheet-like porous body is capable of selective separation of blood cells.
(m) The device for preparing a wound-healing promoting material according to (l), wherein the surface of the sheet-like porous body is capable of selective separation of blood cells.
(n) The device for preparing a wound-healing promoting material according to (m), wherein the sheet-like porous body traps leukocytes and/or platelets more selectively than erythrocytes.
(o) The device for preparing a wound-healing promoting material according to (n), wherein the rate of the sheet-like porous body to trap leukocytes is 50% or higher, and/or the rate of the sheet-like porous body to trap platelets is 50% or higher.
(p) The device for preparing a wound-healing promoting material according to any of (a) to (o), wherein the container is equipped with connecting parts connectable to bags on its inlet and/or outlet side(s).
(q) The device for preparing a wound-healing promoting material according to (p), wherein the container is equipped with a blood-sampling bag on its inlet side and/or a centrifuge bag on its outlet side.
(r) The device for preparing a wound-healing promoting material according to any of (a) to (o), wherein the container is equipped with extracorporeal circulation circuits on its inlet and outlet sides.
(s) The device for preparing a wound-healing promoting material according to any of (a) to (r), which is packaged and sterilized in a sterile bag.
(t) The device for preparing a wound-healing promoting material according to (p), wherein the inlet and the outlet of the container are connected to the exterior and packaged in that state, and the whole apparatus is packaged and sterilized in a sterile bag.

### Brief Description of the Drawing

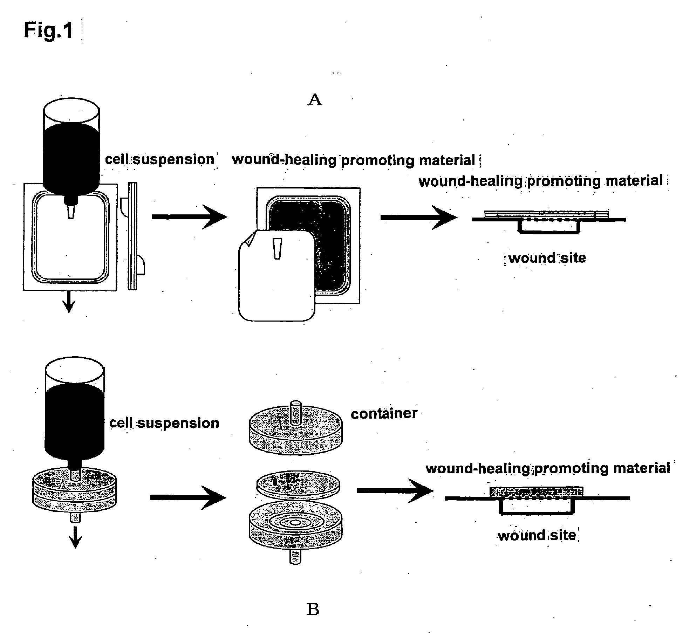

Fig. 1 shows a method for treating a wound site using the wound-healing promoting material according to the present disclosure. Fig. 1A shows a method wherein the wound-healing promoting material is applied to the wound site while allowing the surface of the sheet-like porous body to be exposed from the container. Fig. 1B shows a method wherein the wound-healing promoting material is applied to the wound site by removing the sheet-like porous body from the container.

### Best Mode for Carrying out the Invention

Hereafter, embodiments of the present invention are described in detail.

The sheet-like porous body used in the present invention is a quadrangular or disk-like sheet material. The sheet-like porous body is a porous material which comprises porous portions such as fine pores or interfiber spaces which allows the porous body to trap cells on the surfaces of the porous portions via adsorption or filtration. In general, the surface of a wound site is not always planar but is often irregular. Accordingly, it is effective for a sheet-like porous body to come into close contact with the surface of a wound site according to the shape thereof in order to yield higher wound-healing effects. Thus, it is preferable that the sheet-like porous body according to the present invention is flexible to such an extent that the shape thereof can be altered according to the shape of a wound site. This enables covering of a large wound site of, for example, several centimeters square, with a sheet of porous body without leaving any opening.

The sheet-like porous body used in the present invention is made of a material that can trap at least blood cells, such as leukocytes, from a cell suspension of blood and the like. The sheet-like porous body preferably functions as a filter layer. Specifically, the sheet-like porous body is capable of separation in a manner such that the porous body traps at least blood cells, such as leukocytes and/or platelets, or growth factors but does not trap the liquid portion, when the cell suspension is brought into contact with or passed through the sheet-like porous body. Such separation includes not only size-based separation, but also separation based on cell affinity (e.g., adsorptivity) to the surface of the material.

The sheet-like porous body is preferably capable of selective separation such that a large number of a specific type of blood cells can be trapped. Such selective separation can be realized by adequately selecting the raw material or shape of the porous material. As described below, however, such selective separation can be more selectively controlled by surface treatment of the porous material. The wound-healing promoting material used in the present invention is excellent in terms of, for example, production or secretion of growth factors. Accordingly, a sheet-like porous body that is capable of selectively trapping leukocytes over erythrocytes is particularly preferable. Under such conditions, a target object can be obtained from a smaller amount of cell suspension as the rate of trapping becomes higher. Thus, it is preferred that the rate of trapping leukocytes is 50% or higher.

Examples of forms of the sheet-like porous body include a nonwoven fabric, woven fabric, sponge construct, and a sheet-like bag containing particles. When it is intended to trap leukocytes or platelets, a nonwoven fabric or sponge construct is particularly preferable from the viewpoint of removal efficiency.

The term "nonwoven fabric" used herein refers to a material having a fabric structure made without weaving or knitting a bundle of a layer or more of fibers. Examples of fiber materials that can be used include synthetic, natural, and inorganic fibers. Among them, synthetic fibers mainly composed of hydrophobic polymers, such as polyethylene terephthalate, polybutylene terephthalate, nylon, polypropylene, polyethylene, polystyrene, or polyacrylonitrile are preferably used because of their high cell adhesion.

When the sheet-like porous material is a nonwoven fabric, the average fiber diameter is preferably 0.3 µm to less than 50.0 µm, more preferably 0.5 µm to 40.0 µm, further preferably 0.7 µm to 35.0 µm, still more preferably 1.0 µm to 20.0 µm, and particularly preferably 1.0 µm to 9.0 µm. When the average diameter is smaller than 0.3 µm, fluidity of the blood cell suspension or blood becomes deteriorated upon flushing the same through the nonwoven fabric, which in turn increases pressure loss in the apparatus. In contrast, when the average diameter is larger than 50.0 µm, the rate of trapping leukocytes becomes lowered. If the rate of trapping leukocytes becomes lowered, the distances between cells trapped in the sheet-like porous material become greater in consequence. Accordingly, the average fiber diameter is preferably within the aforementioned range in order for the produced growth factors to more effectively function.

The average diameter of the fibers constituting the nonwoven fabric used in the present invention is determined by, for example, photographing the fibers constituting the nonwoven fabric with a scanning electron microscope, randomly selecting 100 or more fibers, measuring the diameters thereof, and determining the number-average diameter thereof.

The bulk density of the nonwoven fabric used in the present invention is preferably 0.05 g/cm³ to 0.5 g/cm³, more preferably 0.07 g/cm³ to 0.4 g/cm³, and further preferably 0.1 g/cm³ to 0.3 g/cm³. When the bulk density is larger than 0.5 g/cm³, fluidity of the cell suspension or blood becomes deteriorated upon flushing the same through the nonwoven fabric, which in turn increases pressure loss. In contrast, when the bulk density is smaller than 0.05 g/cm³, the rate of trapping cells becomes lowered and the distances between the trapped cells become greater as mentioned above. Accordingly, the bulk density of the fibers is preferably within the aforementioned range in order for the produced growth factors to more effectively function.

The term "sponge construct" used herein refers to a structure having three-dimensional network of connective tissues with continuous open pores. The material of the sponge construct is not particularly limited. Natural polymers, such as cellulose or derivatives thereof, or polymer materials mainly composed of hydrophobic polymers, such as polyolefin, polyamide, polyimide, polyurethane, polyester, polysulfone, polyacrylonitrile, polyethersulfone, poly(meth)acrylate, a butadiene-acrylonitrile copolymer, an ethylene-vinyl alcohol copolymer, polyvinyl acetal, or a mixture thereof, are preferably used because of their high cell adhesion.

The average pore diameter of the sponge construct is preferably 1.0 µm to 40 µm, more preferably 2.0 µm to 35 µm, and further preferably 3.0 µm to 30 µm. When the average pore diameter is smaller than 1.0 µm, blood fluidity becomes deteriorated upon flushing of the cell suspension or blood through the sponge construct, which in turn increases pressure loss in the apparatus. In contrast, when the average pore diameter is larger than 40 µm, the rate of trapping leukocytes becomes lowered. If the average pore diameter of the sponge construct becomes larger, the distances between the cells trapped in the filter layer become greater in consequence. Accordingly, as mentioned above, the average pore diameter of the sponge construct is preferably within the aforementioned range in order for the produced growth factors to more effectively junction.

The average pore diameter of the sponge construct used in the present invention refers to the value obtained by measurement by mercury injection. Based on the measurement by mercury injection (e.g., with the use of Poresizer 9320, Shimadzu Corporation), a graph is made by plotting the derivative values of the pore volumes on a vertical axis and the pore diameters on a horizontal axis. The peak point (mode) is determined as the average pore diameter. The values measured by mercury injection used herein are obtained in the pressure range from 6.89 kPa to 18.27 MPa (1 to 2,650 psia).

The thickness of the sheet-like porous material used in the present invention is 0.01 mm to 3.0 mm, preferably 0.05 mm to 2.5 mm, and more preferably 0.1 mm to 2.0 mm, in order to enhance the wound-healing effects. When the thickness of the sheet-like porous material becomes larger than 3.0 mm, fixation thereof to the wound site becomes difficult. In addition, it becomes difficult for the growth factors generated from leukocytes or platelets to reach the wound site. When the thickness of the sheet-like porous material becomes smaller than 0.01 mm, the mechanical strength thereof becomes deteriorated. The growth factors produced by cells act on such cells themselves (i.e., autocrine) and function more potently in some cases. In other cases, the growth factors produced and spread from the cells in the vicinity act on each other (i.e., paracrine), and the effects thereof may be enhanced between cells. Accordingly, the distance between cells is preferably as small as possible in order for the growth factors to more effectively function. Thus, the thickness of the filter layer is preferably in the aforementioned range. A sheet of the sheet-like porous material having the aforementioned thickness may be used. Alternatively, two or more sheets having a smaller thickness may be laid on top of each other, and the resultant may be used.

Further, the surface of the sheet-like porous material can be coated with a specific polymer or grafted, so that the target object can be selectively adsorbed and trapped. For example, a sheet-like porous material coated with or having thereon a conventional polymer that is likely to adsorb blood cells but adsorbs platelets with difficulty and a sheet-like porous material that is likely to adsorb platelets are treated in that order with blood or a cell suspension. Thus, a sheet-like porous material to which platelets adhere in a more selective manner can be prepared. Such polymers are disclosed in, for example, WO 87/05812, WO03/011924, or WO 03/047655. Also, a ligand that can more selectively adsorb specific components such as blood cells or plasma proteins can be coated or fixed thereon.

The sheet-like porous material can be made of a biodegradable material. Examples of bioadsorbable materials include: polyesters, such as polylactic acid, polyglycolic acid, a lactic acid-glycolic acid copolymer, polymalic acid, and poly-ε-caprolactone; and polysaccharides, such as cellulose, polyalginic acid, chitin, and chitosan. Use of such materials enables the production of a wound-healing promoting material with higher bioadaptability.

The wound-healing promoting material of the present invention must comprise on the surface of the aforementioned sheet-like porous body at least leukocytes. The abundance of blood cells (density) is not particularly limited. In order to enhance the effects as a wound-healing promoting material, however, the blood cell density in a certain volume of porous body is preferably set equal to or higher than the density thereof in the blood. From the viewpoint of the aforementioned intercellular interaction (i.e., involving paracrine), excessively low cell density may cause the production of growth factors to decrease. Accordingly, it is effective to shorten the distance between cells by increasing the cell density. Therefore, it is preferable that the leukocyte density is 6.0x10⁶ cells/cm³ or higher and/or the platelet density is 2.5x10 cells/cm³ or higher on the surface of the sheet-like porous body. The wound-healing effects tend to be improved as the blood cell density increases. When the wound-healing promoting material is prepared via, for example, filtration only, however, the process time may be prolonged or the porous body may be occluded, due to clogging. Thus, the upper limit of the leukocyte density is preferably set at 6.0x10⁸ cells/cm³ or lower, and that of the platelet density is preferably set at 1.0 x 10¹⁰ cells/cm³ or lower.

The aforementioned blood cells that are present on the surface are derived from human peripheral blood, bone marrow, or umbilical cord blood. From the viewpoint of immunological rejection, infection prevention or the like, such blood cells are preferably derived from the blood of the patient who would receive the wound treatment (i.e., autologous blood) or blood having a similar type of the human leukocyte antigen (HLA) (i.e., homologous blood).

The cell suspension that is used to allow the sheet-like porous body to trap these blood cells on its surface and to prepare a wound-healing promoting material contains at least leukocytes. Such suspension may comprise peripheral blood, bone marrow, or umbilical cord blood in that state. It may be a whole blood preparation, a preparation of the segregated blood component, or a cell suspension from which a given cell fraction has been removed in advance. Such cell suspension may comprise, as anticoagulants, citrate, heparins such as common heparin, low-molecular weight heparin or heparinoid, or hydrolase inhibitors such as futhan (FUT), FOY or argatroban. The cell suspension is preferably fresh. The cell suspension is preferably fresh to the extent that the time frame from sampling from the body to filtration through the sheet-like porous body is within 48 hours.

Preferably, blood cells to be present on the surface of the sheet-like porous body include not only immature cells such as stem cells or precursor cells but also a large number of mature cells such as mature leukocytes or platelets, for the following reasons. When a small number of stem cells or precursor cells contained in blood or wounded tissues are proliferated and differentiated for regeneration of wounded tissues, they do not regenerate tissues by themselves. Such immature cells repeat proliferation and differentiation that are optimal for wound healing with a network of mature cells, thereby realizing tissue regeneration.

The wound-healing promoting material used in the present invention can comprise fibroblasts. Fibroblasts can be brought into contact with the sheet-like porous material to be incorporated into the wound-healing promoting material, separately from the blood cell suspension. Alternatively, fibroblasts may be mixed with a cell suspension that contains blood cells in advance and then brought into contact with the sheet-like porous material. Also, fibroblasts may exclusively adhere to other materials (e.g., a filter layer), and the resultant may be combined with the sheet-like porous material to which blood cells have been adhered. It is preferable to incorporate fibroblasts of the same type as those contained in the wound site. This can promote the healing of the wound site.

The wound-healing promoting material used in the present invention can be cultured during its preparation process. The culture liquid is not particularly limited, and any liquid used for common cell culture may be used. Further, culture can be conducted with the addition of the factors that activate blood cells. For example, culture may be conducted following the addition of a platelet activator such as thrombin. Thus, the local concentration of growth factor can be increased.

The wound-healing promoting material used in the present invention can further comprise fibrins. A fibrinogen solution for preparing fibrins is not particularly limited, and a solution commercialized as a pharmaceutical preparation can be used. Also, a liquid that is recovered as drainage from a sheet-like porous material when blood is employed as a blood cell suspension is centrifuged, the centrifugation product can be concentrated, and the concentrate can be used as a fibrinogen solution. When fibroblasts are contained, cells may be previously embedded in fibrins. Also, only cells may be seeded into fibrins later. Incorporation of fibrins into the wound-healing promoting material facilitates the fixation thereof at the wound site. In addition, other growth factors contained in fibrin gel can improve the effects of promoting wound healing.

In order to prepare the wound-healing promoting material used in the present invention, it is necessary that at least leukocytes contained in the cell suspension are trapped on the surface of the sheet-like porous body. The step of trapping may be carried out in the following manner. For example, the sheet-like porous body is spread out and the cell suspension is directly added dropwise thereto for filtration, the sheet-like porous body is held in an adequate filter holder used in a laboratory apparatus and the cell suspension is filtered therethrough (see, for example, Fig. 1B), or the sheet-like porous body is mounted in an openable liquid-tight container, which would be described below in connection with the system, and the cell suspension is then filtered (see, for example, Fig. 1A and Fig. 1B). In the latter two cases, natural filtration may be employed, or forced filtration with the use of a pump or syringe may be employed. In addition to the performance of filtration alone, operation such as maintenance for a given period of time while the cell suspension has been in contact with the porous body may be carried out in combination. This can improve the rate of trapping blood cells on the surface.

When filtration, particularly intermittent filtration, is repeated, cells are disadvantageously activated due to filtration-induced shear stress and growth factors may be disadvantageously released in plasma in the drainage. Accordingly, the cell suspension is preferably filtered in a once-through system, which is simple in terms of preparation and operation and is advantageously carried out within a short period of time. Also, it is preferable to unidirectionally filter a cell suspension. Sometimes leukocytes cannot be trapped in the sheet-like porous body at a sufficient density for some reason, for example, the cell density on one surface of the sheet-like porous body may be different from that on the other surface thereof, and the cell density on the entry side of the filtration system may be higher than that on the other side. In such a case, the surface on the entry side of the filtration system may be brought into close contact with the wound site to more effectively enable the trapped cells to engage in wound healing. Alternatively, cells may be deliberately unevenly distributed to realize such mechanism.

When filtration is carried out in the once-through system, a large amount of cell suspension (one unit, i.e., 400 ml, or more) can be processed, as with the case of leukocyte removal at the time of blood transfusion. When the amount of cell suspension is smaller, however, filtration can be completed within a shorter period of time. Accordingly, the once-through system can be very effective when, for example, preparation is required in parallel with treatment of wounds. The amount of cell suspension to be filtered is preferably set at less than 400 ml. Also, the amount of filtrate per effective filtration area of the sheet-like porous body is preferably set at lower than 10 ml/cm², or the ratio of the amount of filtrate to the volume of the sheet-like porous body is preferably set at lower than 100, since the filtration time becomes shortened.

If an openable liquid-tight container is used, filtration can be easily carried out via extracorporeal circulation. In such a case, the process time becomes relatively long; however, the blood cell density in the sheet-like porous body can be advantageously increased by continuously circulating and filtering a large volume of blood. As described below in connection with the system, a filtration area of 1 to 2 m² can be easily acquired if the sheet-like porous body is wound in a roll and contained in a cylindrical container. Thus, a wound-healing promoting material with a very large area can be obtained from a large volume of blood. In this case, the filtration velocity via extracorporeal circulation is preferably 20 ml/min to 200 ml/min, and the filtration time is preferably 10 to 300 minutes. When filtration is carried out via extracorporeal circulation, the filtration velocity is preferably 20 ml/min to 200 ml/min, more preferably 25 ml/min to 150 ml/min, and further preferably 30 ml/min to 120 ml/min. If the filtration velocity is lower than the lower limit of the aforementioned range, blood is likely to coagulate during extracorporeal circulation, depending on the type of anticoagulant, and the duration of extracorporeal circulation is excessively prolonged, which imposes an excessive burden upon a patient. If the filtration velocity is higher than the upper limit of the aforementioned range, blood sampling occasionally becomes difficult depending on the conditions of a patient's blood vessels, and considerable pressure is applied on the sheet-like porous body, which may disadvantageously promote blood coagulation. The filtration time via extracorporeal circulation is preferably 10 to 300 minutes, more preferably 20 to 250 minutes, and further preferably 30 to 200 minutes. When the filtration time via extracorporeal circulation is shorter than the lower limit of the aforementioned range, a sufficient amount of cells may not be trapped. In contrast, when the filtration time via extracorporeal circulation is longer than the upper limit of the aforementioned range, the burden imposed upon a patient becomes excessive.

Through the above procedure, the wound-healing promoting material used in the present invention that comprises a sheet-like porous body and on its surface at least leukocytes can be produced. This method of production may further comprise a step of washing. Washing may be carried out with the use of an aqueous physiological solution, such as a phosphate buffer or physiologic saline, in the same manner as that employed in the filtration of the cell suspension. Cells, residual blood, and other substances that are not necessary for wound healing can be eliminated via washing. The washed wound-healing promoting material may be kept from drying out and aseptically preserved while being mounted on a holder or in a container or being removed therefrom. The means thereof are not particularly limited.

The present disclosure further relates to a device for preparing the wound-healing promoting material used in the present invention which comprises an openable liquid-tight container equipped with an inlet and an outlet for liquid injection and discharge, wherein a sheet-like porous body is positioned in a manner such that the interior of the container is divided into two sections, and the one end is connected to the inlet and the other end is connected to the outlet.

A liquid-tight container equipped with an inlet and an outlet for liquid injection and discharge refers to a container equipped with an inlet and an outlet capable of injection and discharge (filtration) of the cell suspension or extracorporeal circulation. For example, such container has an appearance such as a conventional planar leukocyte-removal filter device (see, for example, WO 01/091880) or a conventional disk-like leukocyte-removal module (see, for example, WO 99/058172). The interior of the device of the present disclosure is also divided into two sections by a filter (i.e., the sheet-like porous body of the present invention) in order to prevent the liquid that has not yet been filtered from being mixed with the liquid that has been filtered, and the filter is positioned therein in a manner such that the one end is connected to the inlet and the other end is connected to the outlet.

The shape of the container used for the device is not particularly limited. A liquid-tight container that can easily accommodate the sheet-like porous material and that allows the cell suspension to easily flow through the sheet-like porous material may be sufficient. It should be noted that the container is preferably planar if the sheet-like porous material is accommodated in that state. If such material is wound in a roll and then accommodated, a cylindrical container is preferable for easy accommodation. Further, a planar container made of a flexible material may be employed, and the whole planar container is wound in a roll to reduce the size of the container.

The term "openable" used herein refers to the structure of a container, from which the sheet-like porous material that has trapped blood cells can be easily removed, or via which part of the surface on which blood cells have been trapped can be easily exposed. This structure is essential for the device of the present invention. For example, a container body comprises a means of sealing such as a screw, gasket or ground joint, or a container is a laminate of peelable sheets. More specifically, a planar container is preferably a soft device which comprises a container made of flexible resin sheets equipped with a liquid inlet and another container made of flexible resin sheets equipped with a liquid outlet. These containers are welded or made to adhere to each other at the peripheries thereof in a manner such that they encompass the sheet-like porous body. A cylindrical container is preferably a hard device which comprises a header portion equipped with a liquid inlet and the aforementioned means of sealing and another header portion equipped with a liquid outlet and such means of sealing. Such header portions adhere to each end of the cylinder barrel that accommodates the wound sheet-like porous body. The former container is very useful when applying the sheet-like porous body to the wound site in such a manner that part of the sheet-like porous body onto which blood cells have been trapped is exposed from the container constructed with welding or adhesion, as shown in, for example, Fig. 1A. The latter container is suitable for preparation via extracorporeal circulation since a large filtration area can be acquired, regardless of the small size thereof. Thus, such container is suitable to prepare wound-healing promoting materials of large areas or in large amounts.

If the sheet-like porous material onto which blood cells have been trapped can be easily removed from the container or part of the surface onto which blood cells have been trapped can be easily exposed therefrom, an openable means as described above may not be necessary. A container may be composed of a material or structure that can be easily broken entirely or partially from the exterior. Further, a commercialized planar or cylindrical leukocyte-removal filter can be adequately selected and employed.

The device for producing the wound-healing promoting material used in the present invention may comprise a tube with a spike needle at the inlet of the container. This tube is used to aseptically flush the cell suspension and is connected to a blood-sampling bag. A tube alone may be mounted and connected to the bag with an aseptic connector. In order to facilitate the preparation of plasmas or serums using drainage, a centrifuge bag can be connected to the outlet of the container. A common extracorporeal circulation circuit, which is equipped with a blood pump, a portion for blood introduction, a portion for blood reinfusion, or the like, can be further connected to the container.

When the device for producing the wound-healing promoting material used in the present invention is packaged in a sterile bag, a sheet-like porous material is accommodated in a container, this container is packaged in a sterile bag, and the whole device can be packaged in another sterile bag. Thus, the container and the porous material can be aseptically maintained during the time from the flushing of the cell suspension to the actual application of the porous material to the wound site. This can significantly improve the handleability of the device.

wound site can be treated by applying the wound-healing promoting material used in the present invention to the wound site.

Examples of the method for treating a wound site with the wound-healing promoting material include a method wherein the sheet-like porous body is removed from the container and then applied to a wound site (Fig. 1B) and a method wherein the wound-healing promoting material is applied to a wound site while allowing a surface of the sheet-like porous body to be exposed from the container (Fig. 1A). The former method is excellent in terms of sterility of the sheet-like porous body, since the side opposite from the exposed side is covered via the container. Also, the porous body can be prevented from drying out. Accordingly, the former method is particularly suitable in application of the porous material to the surface of the body.

In the method of treating, the surface of the wound-healing promoting material applied to the wound site may be covered and fixed with a protector. In such a case, it is preferable to use a protector made of material having no water permeability in order to prevent the site of treatment, particularly the surface of the wound-healing promoting material, from drying out. Also, use of a material having gas permeability and having no moisture permeability is particularly preferable since use of such material facilitates oxygen delivery to blood cells and maintains cell activity for a longer period of time. Protectors that are commonly used in wound treatment may be employed.

The present invention is hereafter described in greater detail with reference to the following examples.

### Examples

### [Example 1] Production of wound-healing promoting material (human blood)

A sheet-like porous body comprising blood cells on its surface was prepared in the following manner. Peripheral blood was sampled from healthy volunteers using a citrate-phosphate-dextrose (CPD) as an anticoagulant (blood:CPD = 400:56; leukocyte counts: 5,100 cells/µl; platelet counts; 14.4 x 10⁴ cells/µl). A polyethylene terephthalate nonwoven fabric (average fiber diameter: 2.6 µm; thickness: 0.38 mm; bulk density: 0.27 g/cm³, Asahi Kasei Corporation) was punched out into 25-mm-diameter pieces. Two pieces thereof were laid on top of each other and held in a column (model: PP-25, Advantec), and 5 ml of peripheral blood was flushed through the column. Following the blood flushing, 10 ml of phosphate buffer was successively flushed through the column for washing, and the wound-healing promoting material that comprises the sheet-like porous body having blood cells adhering to its surface was obtained.

The above procedure was carried out 2 separate times, and the time required for processing was within 10 minutes in both cases.

The leukocyte counts and the platelet counts in blood before and after processing were measured using a hemocytometer. The rates of trapping leukocytes and platelets with the filter layer of nonwoven fabrics were as shown in Table 1 below.

**Table 1**

| | Rate of trapping leukocytes | Rate of trapping platelets |
|---|---|---|
| First measurement | 74.5% | 83.3% |
| Second measurement | 68.6% | 67.4% |

### [Example 2] Preparation of wound-healing promoting material (human blood)

A sheet-like porous body comprising blood cells on its surface was prepared in the following manner. Peripheral blood was sampled from healthy volunteers using a heparin as an anticoagulant (blood:heparin = 100:1; leukocyte counts: 3,800 cells/µl; platelet counts; 22.5 x 10⁴ cells/µl). A polyethylene terephthalate nonwoven fabric (average fiber diameter: 1.1 µm; thickness: 0.24 mm; bulk density: 0.17 g/cm³, Asahi Kasei Corporation) was punched out into 25-mm-diameter pieces. Two pieces thereof were laid on top of each other and held in a column (model: PP-25, Advantec), and 5 ml of peripheral blood was flushed through the column. Following the blood flushing, 10 ml of phosphate buffer was successively flushed through the column for washing, and the wound-healing promoting material that comprises the sheet-like porous body having blood cells adhering to its surface was obtained.

The above procedure was carried out 2 separate times, and the time required for processing was within 10 minutes in both cases.

The leukocyte counts and the platelet counts in blood before and after processing were measured using a hemocytometer. The rates of trapping leukocytes and platelets with the filter layer of nonwoven fabrics were as shown in Table 2 below.

**Table 2**

| | Rate of trapping leukocytes | Rate of trapping platelets |
|---|---|---|
| First measurement | 74.7% | 72.8% |
| Second measurement | 81.8% | 54.7% |

### [Example 3] Preparation of wound-healing promoting material (mouse blood)

A sheet-like porous body comprising blood cells on its surface was prepared in the following manner. Peripheral blood was sampled from type 2 diabetes mouse models (C57BL/KsJ-db/db Jct, Japan Clea Co., Ltd.) using a heparin as an anticoagulant (leukocyte counts: 5,200 cells/µl; blood:heparin = 100:1). A polylactic acid nonwoven fabric (average fiber diameter: 1.14 µm; thickness: 0.20 mm; bulk density: 0.20 g/cm ³ Asahi Kasei Corporation) was punched out into 13-mm-diameter pieces. Three pieces thereof were laid on top of each other and held in a 5-ml syringe (Terumo Corporation), and 200 µl of mouse peripheral blood was flushed through the syringe. Following the blood flushing, 200 ml of phosphate buffer was successively flushed through the syringe for washing, and the wound-healing promoting material that comprises the sheet-like porous body having blood cells adhering to its surface was obtained.

The above procedure was carried out 2 separate times, and the time required for processing was within 2 minutes in both cases.

The leukocyte counts in blood before and after processing were measured using a hemocytometer. The rate of trapping leukocytes with the filter layer of nonwoven fabrics was as shown in Table 3 below.

**Table 3**

| | Rate of trapping leukocytes |
|---|---|
| First measurement | 68.9% |
| Second measurement | 66.3% |
| Third measurement | 57.5% |

### [Test Example 1] Measurement of the growth rate of fibroblasts

The nonwoven fabric wound-healing promoting material comprising blood cells on its surface, which was prepared in Example 1, was subjected to co-culture with fibroblasts, and the growth rate of fibroblasts was examined.

A multiple well culture plate (Transwell 3452, Corning) with inserts isolated by a membrane with a pore size of 3 µm was used as a culture container.

The nonwoven fabrics (2 pieces) that had been subjected to blood treatment in Example 1 (i.e., the wound-healing promoting material of the present invention having blood cells adhered thereto on its surface) were placed on the top of the insert membrane. The normal diploid fibroblast strains derived from human embryonic lung (human embryonic lung fibroblasts (HEL)) were seeded, at the bottom thereof. The seeded density of the cell was 1 x 10⁵ cells/well.

In accordance with the manufacturer's instructions, sodium pyruvate (ICN), 100x concentrated nonessential amino acid (Cat. #1681049, ICN), and glutamine were added to the Basal Medium Eagle (B 1522, Sigma) to concentrations of 1 mM, 100-fold-dilution, and 2 mM, respectively. Further, fetal calf serum (FCS, StemCell Technologies) to a final concentration of 10% and antibiotic penicillin and streptomycin were added thereto to prepare a culture solution. The volume of the culture solution was 4 ml/well.

As a control, nonwoven fabrics that were not subjected to blood treatment were co-cultured together with HEL.

The above culture product was cultured in an incubator in the presence of 5% CO₂ at 37°C for 4 days, and the number of fibroblasts was then counted. The results are shown in Table 4. Fibroblasts were recovered using a 0.25% Trypsin/EDTA solution (Gibco).

**Table 4**

| | Nonwoven fabric subjected to blood treatment | Nonwoven fabric without treatment |
|---|---|---|
| Cell count | 5.0 x 10⁵ cells/well | 2.0 x 10⁵ cells/well |

As is apparent from Table 4, when culture was conducted using the wound-healing promoting material of the present invention comprising the nonwoven fabrics subjected to blood treatment, which had been prepared in Example 1, the growth rate of fibroblasts was improved as compared with the case where culture was conducted with the use of nonwoven fabrics without blood treatment.

### [Test Example 2] Production of growth factors from wound-healing promoting material

The nonwoven fabric wound-healing promoting material comprising blood cells on its surface, which was prepared in Example 2, was cultured in a culture solution, and the concentrations of various types of growth factors produced from the adhered cells were examined.

A culture dish with a diameter of 35 mm (Asahi Techno Glass Corporation) was used as a culture container.

The nonwoven fabrics (2 pieces) that had been subjected to blood treatment in Example 2 (i.e., the wound-healing promoting material of the present invention having blood cells adhered thereto on its surface) were placed in the container.

Fetal bovine serum (FBS, Gibco) of a final concentration of 2% and antibiotic gentamycin were added to a commercialized culture solution, Dulbecco's Modified Eagle Medium (D-MEM, Gibco), to prepare a culture solution. The volume of the culture solution was 2 ml.

The aforementioned culture product was cultured in an incubator in the presence of 5% CO₂ at 37°C for 48 hours, the culture supernatant was recovered, and the concentrations of VEGF, PDGF-AB, and TGF-β1 were measured as growth factors associated with wound healing. A commercialized ELISA kit (R. & D Systems) was used for measurement of the concentrations.

A culture solution before culturing and plasma which was prepared by recovering blood after filtration through nonwoven fabrics and centrifuging the blood, were employed as a control. The results are shown in Table 5.

**Table 5**

| | VEGF µg/ml | PDGF-AB ng/ml | TGF-β1 ng/ml |
|---|---|---|---|
| First measurement | 635 | 15.1 | 24.5 |
| Second measurement | 452 | 10.6 | 23.7 |
| Culture solution before culturing | 0 | 0 | 0.7 |
| Plasma (First) | 22 | 0.3 | 6.3 |
| Plasma (Second) | 30 | 0.2 | 6.4 |

As is shown in Table 5, a larger amount of growth factors were produced in the culture supernatant resulting from 72-hour culturing of nonwoven fabrics having blood cells on their surfaces than in the culture solution before culturing and in the plasma.

### [Test Example 3]

The wound-healing effects were examined in the following manner with the use of animal models.

The dorsal regions of Type 2 diabetes mouse models (C57BL/KsJ-db/db Jct, Japan Clea Co., Ltd) were shaved under general anesthesia, and they were then subjected to full-thickness surgical wounds using a 6-mm diameter biopsy punch (BP-60F, Kai Industries). Three sites of full-thickness surgical wounds were created per mouse.

Subsequently, the polylactic acid nonwoven fabrics that had been subjected to blood treatment in Example 3 (i.e., the wound-healing promoting material of the present invention having blood cells on its surface), and polylactic acid nonwoven fabrics that had not been subjected to blood treatment were applied to 3 sites of full-thickness surgical wounds. As controls, 3 sites of full-thickness surgical wounds to which no substance had been applied were prepared. These fabrics were fixed with polyurethane wound dressings (Tegaderm, 3M). The control sites were covered with urethane wound dressings only.

The urethane sheets and wound-healing promoting materials were peeled 2 weeks later and the sizes of wounds were measured.

The wound sites and a scale were simultaneously photographed with a digital camera, and the obtained image was analyzed using the ImageJ image analysis software (the National Institutes of Health) to determine the area of the wound. The area 2 weeks after the treatment was calculated while the area of the wound site immediately after the full-thickness skin loss was determined to be 100. The degree of healing was compared, and the results thereof are shown in Table 6.

**Table 6**

| | Mouse 1 | Mouse 2 | Mouse 3 | Average |
|---|---|---|---|---|
| Nonwoven fabric subjected to blood treatment | 22% | 3% | 0% | 8.5 % |
| Nonwoven fabric | 63% | 132% | 294% | 162.9% |
| Without treatment | 92% | 38% | 52% | 60.6% |

As is shown in Table 6, the degree of healing at the site to which the nonwoven fabric that had been subjected to blood treatment was applied was significantly higher than that at other sites. Inflammation was observed at the site to which the polylactic acid nonwoven fabric had been applied, and the wound at such site was likely to be enlarged as compared with that immediately after treatment.

### Industrial Applicability

According to the present disclosure, a wound-healing promoting material that comprises concentrated blood cells can be prepared via a simple procedure within a short period of time. The wound-healing promoting material of the present invention has the effects of promoting cell growth. Therefore, the use of the wound-healing promoting material of the present invention can promote wound healing.

## Claims

1. A material for use as a wound-healing promoting material which comprises a sheet-like porous body having on its surface at least leukocytes.

2. The material for use according to claim 1 which has a fibroblast-proliferating potency.

3. The material for use according to any of claims 1 or 2, wherein the sheet-like porous body has a thickness of 0.01 mm to 3 mm.

4. The material for use according to any of claims 1 to 3, wherein the shape of the sheet-like porous body can be altered in accordance with the shape of the wound site.

5. The material for use according to claim 4, wherein the sheet-like porous body is made of a nonwoven fabric having a fiber diameter of 0.3 µm to 50 µm and a bulk density of 0.05 g/cm³ to 0.5 g/cm³, or a sponge construct having an average pore diameter of 1.0 µm to 40 µm.

6. The material for use according to any of claims 1 to 5, wherein the sheet-like porous body is made of a natural or synthetic polymer.

7. The material for use according to claim 6, wherein the sheet-like porous body is made of a biodegradable material.

8. The material for use according to any of claims 1 to 7, wherein the leukocytes are derived from the peripheral blood, bone marrow fluid, or umbilical cord blood.

9. The material for use according to any of claims 1 to 8, wherein the leukocytes are mature cells.

10. The material for use according to claim 8 or 9, wherein the leukocytes are derived from autologous blood.

11. The material for use according to any of claims 1 to 10, wherein the sheet-like porous body has a leukocyte density of 6.0×10⁶ cells/cm³ or higher.

12. The material for use according to any of claims 1 to 11, wherein the sheet-like porous body comprises fibroblasts incorporated therein.

13. The material for use according to any of claims 1 to 12, wherein the sheet-like porous body comprises fibrins.

14. Use of a sheet-like porous body having on its surface at least leukocytes for the production of a material for use as a wound-healing promoting material.

15. The use according to claim 14, wherein the wound-healing promoting material used in which has a fibroblast-proliferating potency.

16. The use of claim 14 or 15, wherein the sheet-like porous body has a thickness of 0.01 mm to 3 mm.

17. The use according to any of claims 14 to 16, wherein the shape of the sheet-like porous body can be altered in accordance with the shape of the wound site.

18. The use according to claim 17, wherein the sheet-like porous body is made of a nonwoven fabric having a fiber diameter of 0.3 µm to 50 µm and a bulk density of 0.05 g/cm³ to 0.5 g/cm³, or a sponge construct having an average pore diameter of 1.0 µm to 40 µm.

19. The use according to any of claims 14 to 18, wherein the sheet-like porous body is made of a natural or synthetic polymer.

20. The use according to claim 19, wherein the sheet-like porous body is made of a biodegradable material.

21. The use according to any of claims 14 to 20, wherein the leukocytes are derived from the peripheral blood, bone marrow fluid, or umbilical cord blood.

22. The use according to any of claims 14 to 21, wherein the leukocytes are mature cells.

23. The use according to claim 21 or 22, wherein the leukocytes are derived from autologous blood.

24. The use according to any of claims 14 to 23, wherein the sheet-like porous body has a leukocyte density of 6.0×10⁶ cells/cm³ or higher.

25. The use according to any of claims 14 to 24, wherein the sheet-like porous body comprises fibroblasts incorporated therein.

26. The use according to any of claims 14 to 25, wherein the sheet-like porous body comprises fibrins.

## Patentansprüche

1. Material zur Verwendung als wundheilungsförderndes Material, umfassend einen flächigen porösen Körper, der auf seiner Oberfläche wenigstens Leukocyten aufweist.

2. Material zur Verwendung gemäß Anspruch 1, das eine Fähigkeit zur Vermehrung von Fibroblasten aufweist,

3. Material zur Verwendung gemäß einem der Ansprüche 1 oder 2, wobei der flächige poröse Körper eine Dicke von 0,01 mm bis 3 mm aufweist.

4. Material zur Verwendung gemäß einem der Ansprüche 1 bis 3, wobei die Form des flächigen porösen Körpers gemäß der Form der Wundstelle verändert werden kann.

5. Material zur Verwendung gemäß Anspruch 4, wobei der flächige poröse Körper aus einem Vliesstoff mit einem Faserdurchmesser von 0,3 µm bis 50 µm und einer Rohdichte von 0,05 g/cm³ bis 0,5 g/cm³ oder einem Schwammgebilde mit einem mittleren Porendurchmesser von 1,0 µm bis 40 µm besteht.

6. Material zur Verwendung gemäß einem der Ansprüche 1 bis 5, wobei der flächige poröse Körper aus einem natürlichen oder synthetischen Polymer besteht.

7. Material zur Verwendung gemäß Anspruch 6, wobei der flächige poröse Körper aus einem biologisch abbaubaren Material besteht.

8. Material zur Verwendung gemäß einem der Ansprüche 1 bis 7, wobei die Leukocyten aus peripherem Blut, Knochenmarkflüssigkeit oder Nabelschnurblut stammen.

9. Material zur Verwendung gemäß einem der Ansprüche 1 bis 8, wobei die Leukocyten reife Zellen sind.

10. Material zur Verwendung gemäß Anspruch 8 oder 9, wobei die Leukocyten aus autologem Blut stammen.

11. Material zur Verwendung gemäß einem der Ansprüche 1 bis 10, wobei der flächige poröse Körper eine Leukocytendichte von 6,0 × 10⁶ Zellen/cm³ oder mehr aufweist.

12. Material zur Verwendung gemäß einem der Ansprüche 1 bis 11, wobei der flächige poröse Körper Fibroblasten umfasst, die darin enthalten sind.

13. Material zur Verwendung gemäß einem der Ansprüche 1 bis 12, wobei der flächige poröse Körper Fibrine umfasst.

14. Verwendung eines flächigen porösen Körpers, der auf seiner Oberfläche wenigstens Leukocyten aufweist, zur Herstellung eines Materials zur Verwendung als wundheilungsförderndes Material.

15. Verwendung gemäß Anspruch 14, wobei das wundheilungsfördernde Material eine Fähigkeit zur Vermehrung von Fibroblasten aufweist.

16. Verwendung gemäß Anspruch 14 oder 15, wobei der flächige poröse Körper eine Dicke von 0,01 mm bis 3 mm aufweist.

17. Verwendung gemäß einem der Ansprüche 14 bis 16, wobei die Form des flächigen porösen Körpers gemäß der Form der Wundstelle verändert werden kann.

18. Verwendung gemäß Anspruch 17, wobei der flächige poröse Körper aus einem Vliesstoff mit einem Faserdurchmesser von 0,3 µm bis 50 µm und einer Rohdichte von 0,05 g/cm³ bis 0,5 g/cm³ oder einem Schwammgebilde mit einem mittleren Porendurchmesser von 1,0 µm bis 40 µm besteht.

19. Verwendung gemäß einem der Ansprüche 14 bis 18, wobei der flächige poröse Körper aus einem natürlichen oder synthetischen Polymer besteht.

20. Verwendung gemäß Anspruch 19, wobei der flächige porösen Körper aus einem biologisch abbaubaren Material besteht.

21. Verwendung gemäß einem der Ansprüche 14 bis 20, wobei die Leukocyten aus peripherem Blut, Knochenmarkflüssigkeit oder Nabelschnurblut stammen.

22. Verwendung gemäß einem der Ansprüche 14 bis 21, wobei die Leukocyten reife Zellen sind.

23. Verwendung gemäß Anspruch 21 oder 22, wobei die Leukocyten aus autologem Blut stammen.

24. Verwendung gemäß einem der Ansprüche 14 bis 23, wobei der flächige poröse Körper eine Leukocytendichte von 6,0 x 10⁶ Zellen/cm³ oder mehr aufweist.

25. Verwendung gemäß einem der Ansprüche 14 bis 24, wobei der flächige poröse Körper Fibroblasten umfasst, die darin enthalten sind.

26. Verwendung gemäß einem der Ansprüche 14 bis 25, wobei der flächige poröse Körper Fibrine umfasst.

## Revendications

1. Matériau destiné à être utilisé comme matériau favorisant la cicatrisation des plaies qui comprend un corps poreux analogue à une feuille ayant sur sa surface au moins des leucocytes.

2. Matériau destiné à être utilisé selon la revendication 1 qui a une activité de prolifération des fibroblastes.

3. Matériau destiné à être utilisé selon l'une quelconque des revendications 1 ou 2, où le corps poreux analogue à une feuille a une épaisseur de 0,01 mm à 3 mm.

4. Matériau destiné à être utilisé selon l'une quelconque des revendications 1 à 3, où la forme du corps poreux analogue à une feuille peut être modifiée selon la forme du site de la plaie.

5. Matériau destiné à être utilisé selon la revendication 4, où le corps poreux analogue à une feuille est fait d'une étoffe non tissée ayant un diamètre de fibre de 0,3 µm à 50 µm et une masse volumique apparente de 0,05 g/cm³ à 0,5 g/cm³, ou d'une construction de type éponge ayant un diamètre de pore moyen de 1,0 µm à 40 µm.

6. Matériau destiné à être utilisé selon l'une quelconque des revendications 1 à 5, où le corps poreux analogue à une feuille est fait d'un polymère naturel ou synthétique.

7. Matériau destiné à être utilisé selon la revendication 6, où le corps poreux analogue à une feuille est fait d'un matériau biodégradable.

8. Matériau destiné à être utilisé selon l'une quelconque des revendications 1 à 7, où les leucocytes sont dérivés du sang périphérique, du liquide de la moelle osseuse ou du sang du cordon ombilical.

9. Matériau destiné à être utilisé selon l'une quelconque des revendications 1 à 8, où les leucocytes sont des cellules matures.

10. Matériau destiné à être utilisé selon la revendication 8 ou 9, où les leucocytes sont dérivés de sang autologue.

11. Matériau destiné à être utilisé selon l'une quelconque des revendications 1 à 10, où le corps poreux analogue à une feuille a une densité de leucocytes de 6,0 x 10⁶ cellules/cm³ ou plus.

12. Matériau destiné à être utilisé selon l'une quelconque des revendications 1 à 11, où le corps poreux analogue à une feuille comprend des fibroblastes incorporés dans celui-ci.

13. Matériau destiné à être utilisé selon l'une quelconque des revendications 1 à 12, où le corps poreux analogue à une feuille comprend des fibrines.

14. Utilisation d'un corps poreux analogue à une feuille ayant sur sa surface au moins des leucocytes pour la production d'un matériau destiné à être utilisé comme matériau favorisant la cicatrisation des plaies.

15. Utilisation selon la revendication 14, où le matériau favorisant la cicatrisation des plaies qui a une activité de prolifération des fibroblastes.

16. Utilisation selon la revendication 14 ou 15, où le corps poreux analogue à une feuille a une épaisseur de 0,01 mm à 3 mm.

17. Utilisation selon l'une quelconque des revendications 14 à 16, où la forme du corps poreux analogue à une feuille peut être modifiée selon la forme du site de la plaie.

18. Utilisation selon la revendication 17, où le corps poreux analogue à une feuille est fait d'une étoffe non tissée ayant un diamètre de fibre de 0,3 µm à 50 µm et une masse volumique apparente de 0,05 g/cm³ à 0,5 g/cm³, ou d'une construction de type éponge ayant un diamètre de pore moyen de 1,0 µm à 40 µm.

19. Utilisation selon l'une quelconque des revendications 14 à 18, où le corps poreux analogue à une feuille est fait d'un polymère naturel ou synthétique.

20. Utilisation selon la revendication 19, où le corps poreux analogue à une feuille est fait d'un matériau biodégradable.

21. Utilisation selon l'une quelconque des revendications 14 à 20, où les leucocytes sont dérivés du sang périphérique, du liquide de la moelle osseuse ou du sang du cordon ombilical.

22. Utilisation selon l'une quelconque des revendications 14 à 21, où les leucocytes sont des cellules matures.

23. Utilisation selon la revendication 21 ou 22, où les leucocytes sont dérivés de sang autologue.

24. Utilisation selon l'une quelconque des revendications 14 à 23, où le corps poreux analogue à une feuille a une densité de leucocytes de 6,0 x 10⁶ cellules/cm³ ou plus.

25. Utilisation selon l'une quelconque des revendications 14 à 24, où le corps poreux analogue à une feuille comprend des fibroblastes incorporés à l'intérieur.

26. Utilisation selon l'une quelconque des revendications 14 à 25, où le corps poreux analogue à une feuille comprend des fibrines.
